(19) Europäisches Patentamt
European Patent Office
Office européen des brevets

(11) Numéro de publication : **0 006 784**
**B1**

(12) # FASCICULE DE BREVET EUROPÉEN

(45) Date de publication du fascicule du brevet : **19.05.82**

(21) Numéro de dépôt : **79400364.0**

(22) Date de dépôt : **06.06.79**

(51) Int. Cl.³ : **C 07 C149/43**, A 61 K 31/195, A 61 K 31/24// C07C149/415, C07C69/78, C07C121/52

(54) **Nouveaux dérivés de la L-cystéine et leur application comme médicament, ainsi que procédé pour leur préparation.**

(30) Priorité : **22.06.78 FR 7818693**

(43) Date de publication de la demande : **09.01.80 (Bulletin 80/01)**

(45) Mention de la délivrance du brevet : **19.05.82 Bulletin 82/20**

(84) Etats contractants désignés : **DE NL SE**

(56) Documents cités :
**FR - M - 7960**
**FR - M - 8024**
**US - A - 3 137 722**

**Journal of Med. Chem. 1974 - 17 - n° 9, pp. 1 020-1 022.**

(73) Titulaire : **SANOFI, société anonyme**
**40, Avenue George V**
**F-75008 Paris (FR)**

(72) Inventeur : **Maffrand, Jean-Pierre**
**5, rue du Corps Franc Pommiès**
**F-31130 Portet-sur-Garonne (FR)**

(74) Mandataire : **Flechner, Willy et al**
**c/o CABINET FLECHNER 63, Avenue des Champs Elysées**
**F-75008 Paris (FR)**

**EP 0 006 784 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Imprimerie Jouve, 18, rue St-Denis, 75001 Paris, France

Nouveaux dérivés de la L-cystéine et leur application comme médicament, ainsi que procédé pour leur préparation.

La présente invention concerne de nouveaux dérivés de la L-cystéine, un procédé de préparation de ceux-ci et leurs applications en médecine humaine et vétérinaire ainsi qu'en cosmétologie.

Au FR-M 7960, on a jeté sur le papier des composés innombrables par la formule :

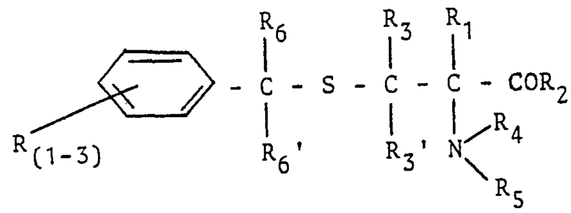

A

dans laquelle R peut être, entre autres, alcanoyle, $R^2$ hydroxy et les autres R de l'hydrogène. Ces produits auraient des propriétés anti-inflammatoires.

Au FR-M 8024, on décrit des composés de formule :

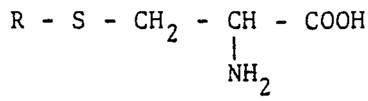

dans laquelle R représente :

soit le radical $(C_6H_5)_3$ C—

soit le radical $(C_6H_5)_2$ CH—

soit le radical $C_6H_5$-$CH_2$—

soit encore le radical HOOC—$(CH_2)_n$—

n étant un nombre entier inférieur ou égal à 4.

Ces composés permettent de lutter contre la séborrhée.

Or, on a maintenant trouvé des composés dont les structures diffèrent de celles décrites à ces deux brevets antérieurs et qui présentent des propriétés mucolytiques inattendues.

Les dérivés de l'invention répondent à la formule développée :

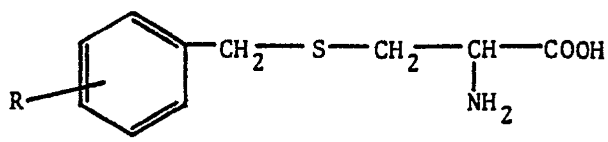

(I)

dans laquelle R représente un groupe alcoxycarbonyle inférieur (notamment en $C_2$ à $C_8$) ou carboxy.

L'invention comprend aussi les sels minéraux ou organiques pharmaceutiquement acceptables des dérivés de formule (I).

L'invention a également pour objet un procédé de préparation des composés de formule (I), caractérisé en ce que l'on condense la L-cystéine de formule :

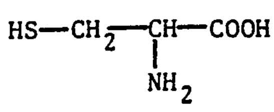

avec un dérivé halogéné de formule :

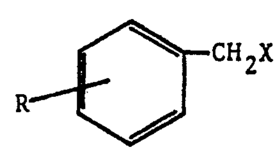

(II)

dans laquelle R représente le groupe cyano ou alcoxycarbonyle et X représente un halogène, tel que le chlore ou le brome par exemple, obtenant ainsi les dérivés de formule (I) dans lesquels R représente le groupe alcoxycarbonyle ou des dérivés cyanés que l'on peut aussi obtenir comme décrit au BSM français 7960 M.

La condensation est effectuée à température ambiante, sous atmosphère protectrice d'azote, en

solution hydroalcoolique, notamment hydroalcanolique inférieur, en présence d'un hydroxyde de métal alcalin, notamment l'hydroxyde de sodium.

Ceux des dérivés (I) pour lesquels R est le groupe carboxy sont obtenus par hydrolyse acide des dérivés (I) correspondants dans lesquels R est le groupe cyano ou alcoxycarbonyle. La réaction s'effectue par chauffage, notamment au reflux, de ces composés dans un acide minéral fort dilué, tel que l'acide chlorhydrique, l'acide bromhydrique ou l'acide sulfurique.

Les dérivés (II) pour lesquels R=CN sont commerciaux. Ceux pour lesquels R est alcoxycarbonyle ont été préparés selon les procédés décrits dans la littérature, par exemple selon P.E. Hanna et al., « J. Med. Chem. », 1974, *17*, 1020.

Les exemples non limitatifs suivants illustrent l'invention.

## Exemple 1

S-(o-méthoxycarbonylbenzyl) cystéine.

On fait barboter de l'azote dans une solution agitée de 8,65 grammes (49,25 mmoles) de chlorhydrate de L-cystéine monohydraté dans 16 $cm^3$ d'eau. On introduit, goutte à goutte, en maintenant la température entre 0 et 5 °C, 49,25 $cm^3$ (98,5 mmoles) de soude 2N, puis une solution de 11,3 grammes (49,25 mmoles) d'α-bromo o-toluate de méthyle dans 100 $cm^3$ de méthanol. Le barbotage d'azote étant maintenu, on poursuit l'agitation à 5 °C pendant une heure (on observe le dépôt d'une phase huileuse sur les parois qui concrétise rapidement en cristaux blancs), puis à température ambiante pendant 20 heures. On évapore à sec et recristallise directement le résidu dans un mélange de 110 $cm^3$ d'eau et 30 $cm^3$ de méthanol. Les cristaux blancs obtenus (9,25 grammes, rendement : 70 %) fondent avec dégagement gazeux à 210 °C.

## Exemple 2

S-(o-carboxybenzyl) cystéine.

On chauffe au reflux pendant 2 heures 30, sous atmosphère d'azote, une solution de 7,65 grammes (28,4 mmoles) de S-(o-méthoxycarbonylbenzyl) cystéine dans 100 $cm^3$ d'acide chlorhydrique 6N. On évapore à sec, dissout le résidu dans un minimum d'eau et porte à un pH de 2,5 à 3 par addition de soude. Les cristaux blancs obtenus sont filtrés et séchés sous vide. F. = 240-245 °C (déc.) (4 grammes, rendement : 55 %).

Ce composé donne un sel monosodique, F. = 252-254 °C, (éthanol-eau), légèrement hygroscopique, très soluble dans l'eau.

## Exemple 3

S-(m-cyanobenzyl) L-cystéine.

Préparé selon le procédé décrit dans l'exemple 1, à partir du bromure de m-cyanobenzyle.

Méthanesulfonate, cristaux blancs, F. = 130-135 °C, (éthanol-isopropanol), légèrement hygroscopique, rendement : 44,5 %.

## Exemple 4

S-(p-cyanobenzyl) L-cystéine.

Préparé selon le procédé décrit dans l'exemple 1, à partir du bromure de p-cyanobenzyle.

Base : cristaux blancs, F. = 171 °C, (éthanol-isopropanol), rendement : 10 %.

## Exemple 5

Préparé selon le procédé décrit dans l'exemple 2 par chauffage au reflux de la S-(m-cyanobenzyl) L-cystéine (exemple 3) dans l'acide chlorhydrique 12N.

Base : cristaux rosés, F. >260 °C, (diméthyl-formamide), rendement : 64 %.

## Exemple 6

S-(p-carboxybenzyl) L-cystéine.

Préparé selon le procédé décrit dans l'exemple 2 par chauffage au reflux de la S-(p-cyanobenzyl) L-cystéine (exemple 4) dans l'acide chlorhydrique 12N.

Base : cristaux rosés, F. >260 °C, (diméthylformamide), rendement : 84 %. Ce composé donne un sel monosodique, F. >260 °C, (éthanol-eau), légèrement hygroscopique, très soluble dans l'eau.

L'expérimentation toxicologique et pharmacologique qui est rapportée ci-après a montré que les dérivés de l'invention possédaient des propriétés mucolytiques remarquables, tout en étant totalement dépourvues de toxicité.

L'invention a donc encore pour objet un médicament possédant en particulier une activité

mucolytique, caractérisé en ce qu'il contient, à titre de principe actif, un dérivé de formule (I) ou un sel pharmaceutiquement acceptable de celui-ci.

En outre, il a été vérifié que les dérivés de l'invention sont doués de propriétés trophiques envers la peau et les phanères et jouent un rôle protecteur envers l'épiderme, les ongles et les cheveux.

I. Etude toxicologique.

Les composés de l'invention bénéficient d'une très faible toxicité aiguë. Ainsi la DL 50/24h/kg de poids corporel, déterminée chez la souris, selon la méthode de Miller et Tainter, pour la voie orale est supérieure à 3 grammes.

En outre, les essais effectués chez différentes espèces animales, sur les toxicités aiguë, chronique, sub-chronique et retardée n'ont pas permis de mettre en évidence une quelconque réaction locale ou générale, une perturbation dans l'évolution pondérale des animaux, une perturbation dans les contrôles biologiques régulièrement effectués, une anomalie dans les examens microscopiques et macroscopiques des organes prélevés.

II. Etude pharmacologique.

Cette étude de l'activité mucolytique des dérivés de l'invention a été effectuée selon la méthode décrite par André Quevauvilliers, Suzanne Garcet et Vu Ngcoc Huyen (Produits et Problèmes Pharmaceutiques, 27, 267-280, 1972) qui consiste à provoquer chez le rat, par inhalation de gaz irritants, des lésions de la muqueuse respiratoire et, du même coup, une hypersécrétion bronchique.

Ainsi, les rats sont soumis à l'inhalation d'anhydride sulfureux ($SO_2$) à la concentration de 300 ppm, 4 heures par jour, 5 jours par semaine, pendant 8 semaines. Pendant les 4 premières semaines, les animaux ne reçoivent aucun traitement.

Les 4 semaines suivantes, les animaux du lot A (témoin), continuant à ne recevoir aucun traitement, on administre aux animaux du lot B (témoin traité) 500 mg/kg de S-carboxyméthyl-cystéine et aux animaux du lot C (traité) 500 mg/kg du dérivé à tester.

24 heures après l'arrêt du traitement, les animaux sont sacrifiés et on prélève les poumons et les bronches.

Après avoir injecté une solution de bleu Alcian dans la bronche principale, des coupes macroscopiques sont effectuées. L'examen, à l'aide de la loupe binoculaire, permet de se rendre compte de l'état sécrétoire de la muqueuse de l'arbre bronchique. L'hypersécrétion, quand elle existe, obstrue partiellement ou complètement les bronches principales et secondaires. Quand elle est totale, l'obstruction peut être constituée d'un bouchon compact, d'amas nodulaires et d'amas bulbeux.

Les résultats sont rassemblés dans le tableau ci-après, les valeurs exprimées étant des moyennes réalisées à l'intérieur de chaque lot (pourcentages).

Les résultats de ces études ont mis en évidence la bonne tolérance et la faible toxicité des dérivés de l'invention, en même temps que leur remarquable activité mucolytique qui les rendent très utiles en médecine humaine et vétérinaire.

Le médicament de l'invention peut donc être présenté, pour l'administration orale, sous forme de comprimés, comprimés dragéifiés, capsules et sirop. Il peut aussi être présenté pour l'usage externe, sous forme de gouttes ou de pulvérisations nasales.

Chaque dose unitaire contient avantageusement de 0,050 gramme à 1,0 gramme de principe actif, les doses administrables journellement variant de 0,050 gramme à 3 grammes selon la gravité de l'affection traitée et l'âge du patient. Pour l'usage externe, les solutions utilisées contiennent avantageusement de 2 à 5 % de principe actif.

On donnera, ci-après, à titre d'exemple non limitatif, quelques formulations pharmaceutiques du médicament de l'invention.

Comprimés dragéifiés.

Dérivé n° 2 : 0,500 gramme.

Excipient : cellulose, carboxyméthyl cellulose sodique, stéarate de magnésium, talc, acide stéarique, colophane, essence de térébenthine, gomme laque, gélatine, sucre, talc, cire blanche, oxyde de titane, érythrosine.

Gouttes nasales.

Dérivé n° 5 : 2,00 grammes.

Excipient aqueux q.s.p. : 100 millilitres.

Pour leur utilisation en cosmétique, les dérivés de l'invention peuvent être mélangés aux véhicules solides ou liquides adéquats, pharmaceutiquement acceptables, et présentés sous forme de crème, pommade ou lotion.

Les études qui viennent d'être rapportées ont montré la remarquable action mucolytique des dérivés de l'invention qui représentent une thérapeutique de choix des affections de la muqueuse respiratoire. Par une action de lyse sur les sécrétions pathologiques, ils facilitent l'expectoration et désencombrent les voies respiratoires. D'autre part, ils contribuent à rétablir la sécrétion physiologique du mucus et ainsi à protéger les voies respiratoires.

Ils sont indiqués en pneumologie dans les bronchites chroniques, les trachéo-bronchites, les bronchorhées et en oto-rhino-laryngologie dans les otites, sinusites, pharyngites et rhinopharyngites.

| | Bouchon compact | Amas nodulaire | Amas bulbeux | Obstruction complète | Obstruction partielle | Obstruction totale et partielle |
|---|---|---|---|---|---|---|
| $SO_2$ | 51 | 8 | 3 | 63 | 9 | 72 |
| S-carboxy-méthyl-cystéine | 10 | 2 | | 12 | 8 | 20 |
| Dérivé 1 | 7 | 3 | | 10 | 8 | 18 |
| Dérivé 2 | 8 | 2 | 1 | 11 | 8 | 19 |
| Dérivé 5 | 10 | 2 | | 12 | 5 | 17 |
| Dérivé 6 | 8 | 3 | | 11 | 6 | 17 |

**Revendications**

1. Dérivés de la L-cystéine de formule :

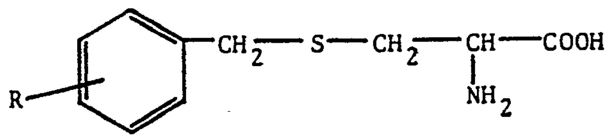

dans laquelle R est alcoxycarbonyle inférieur ou carboxy et leurs sels.

2. La S-(o-méthoxycarbonylbenzyl) cystéine.

3. La S-(o-carboxybenzyl) cystéine.

4. La S-(m-carboxybenzyl) cystéine.

5. La S-(p-carboxybenzyl) cystéine.

6. Médicament notamment mucolytique, caractérisé en ce qu'il comprend, à titre de principe actif, un dérivé suivant l'une des revendications 1 à 5.

7. Procédé de préparation de dérivés de la L-cystéine de formule :

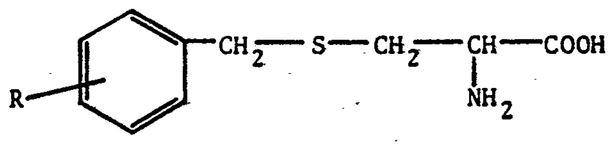

et de leurs sels, caractérisé en ce qu'il consiste à condenser la L-cystéine de formule :

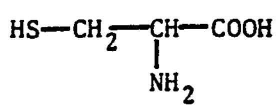

sur un composé halogéné de formule :

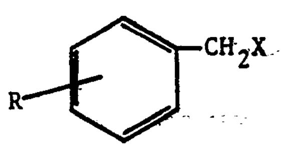

dans laquelle R est le groupe cyano ou alcoxycarbonyle et X est un halogène, pour obtenir les dérivés dans lesquels R est alcoxycarbonyle ou un dérivé cyané, et pour obtenir les dérivés dans lesquels R est carboxy à hydrolyser en milieu acide minéral les dérivés alcoxycarbonylés ou cyanés et, pour obtenir les sels, à salifier l'acide par une base.

**Claims**

1. L-cysteine derivatives of formula :

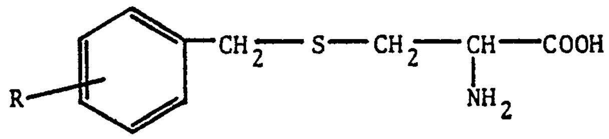

in which R is lower alkoxycarbonyl or carboxy and their salts
2. S-(o-methoxycarbonylbenzyl) cysteine
3. S-(o-carboxybenzyl) cysteine
4. S-(m-carboxybenzyl) cysteine
5. S-(p-carboxybenzyl) cysteine
6. Medicament, especially mucolytic, characterized in that it contains as active ingredient a derivative according to one of claims 1 to 5.
7. Process for the preparation of L-cysteine derivatives of formula :

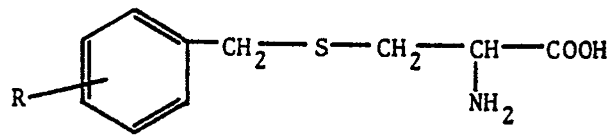

and their salts, characterised in that the L-cysteine of formula :

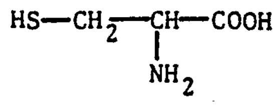

is condensed with a halogenated compound of formula :

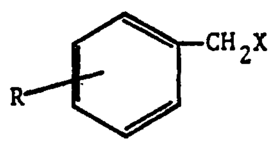

in which R represents the cyano or alkoxycarbonyl group and X represents a halogen, thus obtaining the derivatives in which R is alkoxycarbonyl or a cyano derivative, and, to obtain the derivatives in which R is carboxy, the cyanated or alkoxycarbonylated derivatives are hydrolysed in a mineral acid medium and, to obtain the salts, the acid is salified with a base.

**Ansprüche**

1. Derivate des L-Cysteins der Formel :

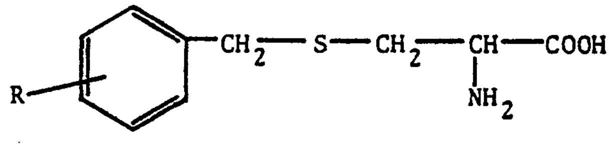

in der R für einen kurzkettigen Alkoxycarbonyl- oder Carboxyrest steht, sowie ihre Salze.
2. S-(o-Methoxycarbonylbenzyl) cystein.
3. S-(o-Carboxybenzyl) cystein.
4. S-(m-Carboxybenzyl) cystein.
5. S-(p-Carboxybenzyl) cystein.
6. Medikament, insbesondere muculytisches Medikament, dadurch gekennzeichnet, daß es als wirksame Komponente ein Derivat nach einem der Ansprüche 1 bis 5 enthält.
7. Verfahren zur Herstellung von Derivaten des L-Cysteins der Formel :

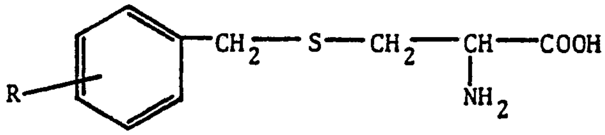

und ihrer Salze, dadurch gekennzeichnet, daß man L-Cystein der Formel :

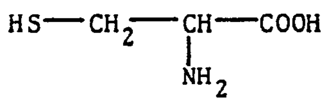

mit einer Halogenverbindung der Formel :

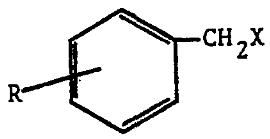

in welcher R ein Cyano- oder Alkoxycarbonylrest und X ein Halogenatom ist, zu Derivaten, in denen R ein Alkoxycarbonylrest ist oder zu Cyanoderivaten kondensiert und daß man zur Herstellung von Derivaten der angegebenen Formel, in der R für einen Carboxyrest steht, die Alkoxycarbonyl- oder Cyanoderivate mit einer Mineralsäure hydrolysiert und daß man zur Gewinnung von Salzen die Säuren mit einer Base in Salze überführt.